# EUROPEAN PATENT APPLICATION

(11) **EP 1 845 378 A1**
(43) Date of publication of application: **17.10.2007**
(21) Application number: 05714754.8
(22) Date of filing: 02.02.2005
(51) Int. Cl.: G01N 33/576, G01N 33/543

(54) **A KIT FOR DETECTING THE ANTIBODY OF HCV AND ITS PREPARING METHOD**

(71) Applicant: Cui, Peng, NanShan District Shenzen, Guangdong 518052 (CN)
(72) Inventor: Cui, Peng, NanShan District Shenzen, Guangdong 518052 (CN)
(74) Representative: Bonnetat, Christian
(86) International application number: PCT/CN2005/000149
(87) International publication number: WO 2006/081701

(57) **Abstract**

A kit and its preparing method concerning dual-antigen sandwich method are used for detecting the antibody against HCV, and its detecting mode is 'carrier-first antigen-antibody against HCV to be detected-second antigen-marker- distinguishable signal'. The kit ant its preparing method characterize in that the second antigen is the complex of a HCV and a tag.

SEQUENCE LIST

<110> Cuj Peng

<120> A Hepatitis C virus antibodics diagnostic reagent kit and its preparation method

<160> 4

<170> PatentIn version 3.3

<210> 1
<211> 270
<212> DNA
<213> Hepatitis C virus(HCV)

<220>
<223> The whole sequence of HCV gene coding a.a. 1-90

<400> 1
ATGAGCACGA ACCCTAAACC TCAAAGAAAA ACCAAACGTA ACACCAACCG CCGCCCACAG 60
GACGTCAAGT TCCCGGGCGG TGGTCAGATC GTTGGTGGAG TTTACCTGTT GCCGCGCAGG 120
GGCCCCAGGT TGGGTGTGCG CGCGACTAGG AAGACTTCCG AGCGGTCACA ACCTCGTGGA 180
AGGCGACAAC CTATCCCCAA GGCTCGCCGT CCCGAGGGCA GGACCTGGGC TCAGCCCTGG 240
TACCCTTGGC CCCTCTATGG CAATGAGGGC 270

<210> 2
<211> 834
<212> DNA
<213> Hepatitis C virus(HCV)

<220>
<223> The whole sequence of HCV gene coding a.a. 1196-1473

<400> 2
ATCCCTGTGG AGAACCTAGA AACTACCACG CGGTCTCCGG TTTTCACAGA CAACTCATCC 60
CCCCCGGCCG TACCGCAGAC ATTCCAAGTG GCCCATCTAC ACGCTCCTAC TGGCAGCGGC 120
AAGAGCACTA AAGTGCCGGC TGCATATGCG GCCCAAGGGT ACAAGGTGCT CGTCCTGAAC 180
CCGTCCGTTG CCGCCACCTT AGGTTTTGGG GCGTATATGT CTAAGGCACA TGGTATCGAC 240
CCTAGCGTCA GGACCGGGGT AAGGACCATC ACCACGGGTA GCCCCATCAC GTACTCCACC 300
TATGGCAAGT TCCTTGCTGA TGGTGGTTGC TCTGGGGGTG CCTATGACAT TATAATATGT 360
GATGAGTGCC ATTCAACTGA CTCGACTACT ATCTTGGGCA TCGGCACGGT CCTGGACCAA 420
GCGGAGACGG CTGGAGCGCG GCTTGTCGTG CTCGCCACCG CTACGCCTCC GGGATCGGTC 480
ACCGTGCCAC ATCCCAACAT CCAGGAGGTG GCCCTGTCCA ATACTGGAGA GATCCCCTTC 540
TATGGTAAAG CCATCCCCAT CGAGGCCATC AGGGGGGGAA GGCATCTCAT TTTCTGCCAC 600
TCCAAGAAGA AGTGTGACGA GCTCGCTGCA AAGCTATCAT CCCTCGGACT CAACGCTGTG 660
GCGTACTACC GGGGTCTTGA TGTGTCCGTC ATACCATCTA GCGGAGACGT CGTTGTCGTG 720
GCAACAGACG CTCTAATGAC GGGCTTTACC GGCGACTTTG ACTCAGTGAT CGACTGTAAC 780
ACGTGCGTCA CCCAGACAGT CGATTTCAGC CTTGACCCTA CCTTCACCAT TGAG 834

<210> 3
<211> 69

<212> DNA
<213> 未知

<220>
<223> The sequence of gene coding biotin receptor protein

<400> 3
ATGGCTGGTG GTTTAAACGA CATCTTTGAA GCTCAGAAAA TCGAATGGCA TGAAGACACC 60
GGTGGCTCT 69

<210> 4
<211> 966
<212> DNA
<213> Escherichia coli

<220>
<223> The sequence of gene coding biotin-[acetyl-CoA carboxylase] synthetase BirA

<400> 4
ATGAAGGATA ACACCGTGCC ACTGAAATTG ATTGCCCTGT TAGCGAACGG TGAATTTCAC 60
TCTGGCGAGC AGTTGGGTGA AACGCTGGGA ATGAGCCGGG CGGCTATTAA TAAACACATT 120
CAGACACTGC GTGACTGGGG CGTTGATGTC TTTACCGTTC CGGGTAAAGG ATACAGCCTG 180
CCTGAGCCTA TCCAGTTACT TAATGCTAAA CAGATATTGG GTCAGCTGGA TGGCGGTAGT 240
GTAGCCGTGC TGCCAGTGAT TGACTCCACG AATCAGTACC TTCTTGATCG TATCGGAGAG 300
CTTAAATCGG GCGATGCTTG CATTGCAGAA TACCAGCAGG CTGGCCGTGG TCGCCGGGGT 360
CGGAAATGGT TTTCGCCTTT TGGCGCAAAC TTATATTTGT CGATGTTCTG GCGTCTGGAA 420
CAAGGCCCGG CGGCGGCGAT TGGTTTAAGT CTGGTTATCG GTATCGTGAT GGCGGAAGTA 480
TTACGCAAGC TGGGTGCAGA TAAAGTTCGT GTTAAATGGC CTAATGACCT CTATCTGCAG 540
GATCGCAAGC TGGCAGGCAT TCTGGTGGAG CTGACTGGCA AAACTGGCGA TGCGGCGCAA 600
ATAGTCATTG GAGCCGGGAT CAACATGGCA ATGCGCCGTG TTGAAGAGAG TGTCGTTAAT 660
CAGGGGTGGA TCACGCTGCA GGAAGCGGGG ATCAATCTCG ATCGTAATAC GTTGGCGGCC 720
ATGCTAATAC GTGAATTACG TGCTGCGTTG GAACTCTTCG AACAAGAAGG ATTGGCACCT 780
TATCTGTCGC GCTGGGAAAA GCTGGATAAT TTTATTAATC GCCCAGTGAA ACTTATCATT 840
GGTGATAAAG AAATATTTGG CATTTCACGC GGAATAGACA AACAGGGGGC TTTATTACTT 900
GAGCAGGATG GAATAATAAA ACCCTGGATG GGCGGTGAAA TATCCCTGCG TAGTGCAGAA 960
AAATAA 966

## Description

### Technical Field:

The present invention relates to immunodiagnosis. In particular, this invention relates to HCV antibody diagnostic kit and its preparation method.

### Background:

Hepatitis C is a serve hepatic disease caused by hepatic C virus (HCV). About 170 million patients are infected with HCV over the world and in China the number has exceeded 40 million. 50-85% of HCV-infected individuals subsequently develop to chronic hepatitis and 10-15% of them will be deteriorated to hepatocirrhosis. So HCV brings great harm to human's healthy and heavy burden to our society.

HCV Diagnosis made much progress since the HCV gene was cloned by Choo et al in 1989. Now there are 3 main methods for Diagnosis of HCV; detecting the HCV RNA by polymerase chain reaction (PCR); detecting the HCV antigens using monoclonal antibody; detecting the HCV antibodies with ELISA and Western blotting.

ELISA for detecting HCV antibodies have developed into the third generation. The primitive Elisa-1 kit was developed by Ortho Diagnosis Company (U.K), and the antigen was HCV NS4 recombinant antigen called C100-3 which was provide by Chiron Company (U.S.A), but its sensitivity and specificity are not very satisfied. Thereafter Elisa-2 kits came out to be substitute for Elisa-1 kits. Ortho Diagnosis Company and Abbott Diagnosis Company (U.S.A) used CORE, NS3 and NS4 antigens in the Elisa-2 kits and found its sensitivity and specificity improved in comparison with the Elisa-1 kits. Clinic use showed that positive rate was 92~95% when Elisa-2 kits were applied in chronic hepatitis patients. In addition, the average window phase of serum HCV seropositive conversion was about 10 weeks which was much shorter than the Elisa-1 kits (16weeks). Elisa-3 kits were developed by Ortho Diagnosis Company (U.K), Abbott Diagnosis Company (U.S.A) and Sanofi Diagnosis Company (France) in the world. Antigens used in these kits included CORE, NS3, NS4 and NS5. Detection of HCV in blood donors and subjects who was susceptible to be infected using ELISA-3 kits showed that the sensitivity increased to 97% and the average window phase of serum HCV seropositive conversion was about 7-8 weeks.

Elisa can be automation, is easy for operation and analysis& saving data, moreover, it presents good reproducibility and the cost is relatively low. Therefore, commercial reagents for detectting HCV antibody are mainly Elisa-2 and Elisa-3 kits nowadays. Althought improved many, the Elisa-3 kit still has many disadvantages, such as false negative, false positive, and uncertain results. It may due to inherence defects of the indirect method in methodology. The following is the shortcoming:
1. Poor specificity. We know the second antibody (SA) does not specially recognize the primitive antibody in the direct method, which leads to low specificity.
2. Low sensitivity. Because of poor specificity, the concentration of coating antigen and labeled SA must be low, thus the sample volume has to be reduced and the multiples dilution should be higher, which results to more lower sensitivity.
3. Long window period. The common SA is antibody to anti-lgG, which can only detect the IgG in HCV antibodies and be unable to diagnose other antibodies especially the IgM antibody emerged in the early period. That cause low sensitivity and prolong the window period.

At present, 70% of Individuals developing post-transfusion hepatitis were those infected with HCV after blood transfusion. Among HCV carriers 80~90% are infected after blood transfusion. This serious phenomenon suggests that our available diagnostic kits for detecting HCV antibodies have much deficiency, so kits with high sensitivity and specifctiy are requested.

Double antigens sandwiched assay use labeled antigen as a substitution for labeled SA in the indirect method, so disadvantages due to indirect method can be solved. We know sandwiched ELISA is commonly applied method -nowadays, but for detecting HCV antibody, there is no kits using the method. There are two main reasons:

### 1. The activity loss following labeling the HCV protein

Presently to conjugate the antigen with main label such as peroxidase horseradish (HRP) is the dominant enzyme labeling method, but there are some problems when this method is applied for HCV antigen. First, the NS3 antigen as the main antigen epitope area of HCV, is the one strongly depending on its conformation, and when labeled by a "flexible" label with high molecular weight such as peroxidase horseradish or alkaline phosphatase it varied greatly in conformation, and its active epitopes are difficult to expose. All those decrease the activity of labeled NS3 antigen substantially. The main active site in HCV CORE antigen epitope area contains lots of Lysine which are critical to the activity of CORE, and the free amino-group in the side chain is used as the target for HRP labeling by NalO4 oxidization. So the activity of CORE antigen is decreased after labelling, and as a result the sensitivity of the kits produced as the above-mentioned are even less than those of indirect methods, leading to the mis-diagnosis seriously.

### 2. The high background

The CORE antigen has very powerful bind capacity of in vivo or homologous and heterogenous in vitro (Matsumoto, M. etc., Virology, 218:P43-51,1996; Kunkel, M. etc., Virology, 294: P239-245, 2002; Majeau, N. etc., Journal of General Virology, 85: P971-981, 2004)). The labeled antigen (the second antigen) in the sandwiched method of double antigens can bind homologously with the coating antigen (the primary antigen) which leads to the high background of diagnostic kit.

By now there are lots of reports about double antigen sandwich assay detection reagent for HCV antibody abroad, but there are some defects in each detection reagent, and the main points are as follow:

In patent US6096319, US6270960, US6306579, though double antigen sandwich assay and some method for solving the mismatching of disulfide in NS3 antigen is talked, they never talk about how to solve the mismatching disulfide in enzyme labeled HCV NS3 antigen, peroxidase horseradish especially.

In patent US6613530, CN 1548958A. the described double antigen sandwich assay for HCV antigen never mentioned the way to solve the mismatching disulfide in coated NS3 antigen or in enzyme labeled NS3 antigen.
In patent US6630298, the kit detecting HCV antigen and HCV antibody simultaneously by double antigen sandwich assay. Though 1,4-Dithiothreitol was mentioned, DTT is only used as denaturing reagent and point out DTT can destroy the conformation of the epitope of NS3. They didn't mention the function of Dithiothreitol for breaking the mismatching disulfide and increasing the antigenicity for recombinant NS3 antigen.

This invent utilize double antigen sandwich assay to research a HCV antibody detection kit. We not only overcome many faults of indirect method in methodology but also solve many technique problems in present double antigen sandwich assay kit, and develop HCV antibody detection kit by double antigen sandwich assay for clinical detection. The kit could provide a much powerful choice for HCV detection.

### OBJECTIVES OF THE INVENTION

It is the objective of the present invention to solve the problem of the high background caused by CORE antigen in the HCV double antigen sandwich kit and solve the problem of activity loss after labeling HCV protein, and furthermore to combine biotin-avidin magnifying system to provide a new double-antigen sandwich test Kit for HCV antibodies, whose sensitivity and specificity are much better than present commercial reagents.

### SUMMARY OF THE INVENTION

The present invention provides a HCV antibodies test kit. The kit completes detection in the form of 'support-primary antigen-HCV antibodies to be detected-secondary antigen-label-detectable signal', wherein-secondary antigens take place one or more association reaction with label. The kit is characterized in that said secondary antigens are conjugates of HCV protein and tag.

In an embodiment of the present invention, said the tag is peptide tag or non-peptide tag.

In a further embodiment of the present invention, said non-peptide tag is chemical compound, hapten, vitamin, steroid, dyestuff, hormone, antibiotic, nucleic acid or conjugate of all of the above component with peptide or protein.

in the preferred embodiments of the present invention, said chemical compound is dinitrophenol, bromodeoxyuridine; vitamin is biotin or derivatives thereof; steroid is Digoxin; dyestuff is Acridinium Ester, Rhodamine, Dansyl Chloride, Dihydroxyfluorane, Oregon Green, Lucifer yellow, Alexa Fluor, Cascade Blue.

In a further preferred embodiment of the present invention, said non-peptide tag is biotin or derivatives thereof, conjugate of biotin or derivatives thereof with peptide or protein.

In a further embodiment of the present invention, said peptide tag is peptide or protein containing His Tag, T7 Tag, S Tag, Flag Tag, HA Tag or HCV peptide fragment.

In the preferred embodiments of the present invention, when said peptide tag is heterologous protein, its molecular weight is smaller than 30KD, preferable smaller than 20KD, and furthermore preferable smaller than 15KD, and furthermore preferable smaller than 10KD, and furthermore preferable smaller than 5KD.

In the preferred embodiments of the present invention, said heterologous protein peptide tag is at -N-terminal of the HCV protein, its molecular weight is smaller than 5KD; when said peptide tag is at C-terminal, its molecular weight is smaller than 20KD.

In an embodiment of the present invention, said primary antigen and secondary antigen contain protein segments of NS3 and CORE. And NS3 segments are any segments from a.a.1201±5 to a.a.1465±8, which can have mutation while keeping original antigenicity. CORE protein are segments which contain a. a. 10-17 complete sequence, and furthermore are segments which contain a.a.7-21 complete sequence, and furthermore are segments which contain a.a.6-23 complete sequence; moreover said segments do not contain a.a.29-90 complete sequence segments, and furthermore do not contain a.a.29-70 complete sequence segments, and furthermore do not contain a.a.29-59 complete sequence segments, and furthermore do not contain a.a.32-48 complete sequence segments. The above protein segments can have mutations while keeping original antigenic activity and binding ability.

In the preferred embodiments of the present invention, said CORE segment of primary antigen is a.a.2-59; said CORE segment of secondary antigen is a.a.1-28; said NS3 segments of first and secondary antigen are both a.a.1201-1465.

In an embodiment of the present invention, the binding partner of said tag is tag antibody or tag acceptor.

In a further embodiment of the present invention, when choosing biotin or derivatives thereof, conjugate of biotin or derivatives thereof with peptide or protein to be tag, their binding partners are avidin, streptavidin, neutron-streptavidin, their analogue, antibodies of antibiotic or derivatives thereof; when choosing a peptide to be tag, its binding partner is monoclonal antibody or polyclonal antibody against it.

The present invention also provides a method for prepared said kit, which comprise steps of conjugating HCV protein with tag. The method includes:
1. Biotinylation of HCV protein: including enzymatic biotinylation in vivo, biotinylation in vitro, indirect biotinylation, biotinylation of amino acid;
2. Chimeric expression of HCV protein and peptide.

In a further embodiment of the present invention, said enzymatic biotinylation in vivo method is:
The Chimeric gene encoding HCV protein and biotin acceptor protein (short for X) and the gene encoding biotin-[acetyl-CoA carboxylase] synthase (short for Y) are co-expressed in the host, making particular lysine residue of the protien encoded by the chimeric gene X associated to biotin via enzymatic reaction in vivo with expressed biotin-[acetyl-CoA carboxylase] synthase in host. The particular expression method comprise:
   1) Polycistron expression;
   2) Cotransformation expression;
   3) Poly-promotor expression;
   4) Monocistronic expression;
   5) Co-expression with host biotin-[acetyl-CoA carboxylase] synthase gene.

In other embodiments of the present invention, said biotinylation in vitro is performed by coupling activated biotin or enzymatic reaction in vitro to complete the biotinylation of HCV protein.

In an embodiment of the present invention, said biotin acceptor protein are biotin carboxyl-carrier protein family or domain segment thereof, -SEQ ID NO.3 polypeptide or domain analogs thereof.

In an embodiment of the present invention, said biotin-[acetyl-CoA carboxylase] synthase is enzyme whose class number is EC6.3.4.15 or domain segments and domain analogs thereof.

### DESCRIPTION OF THE FIGURES

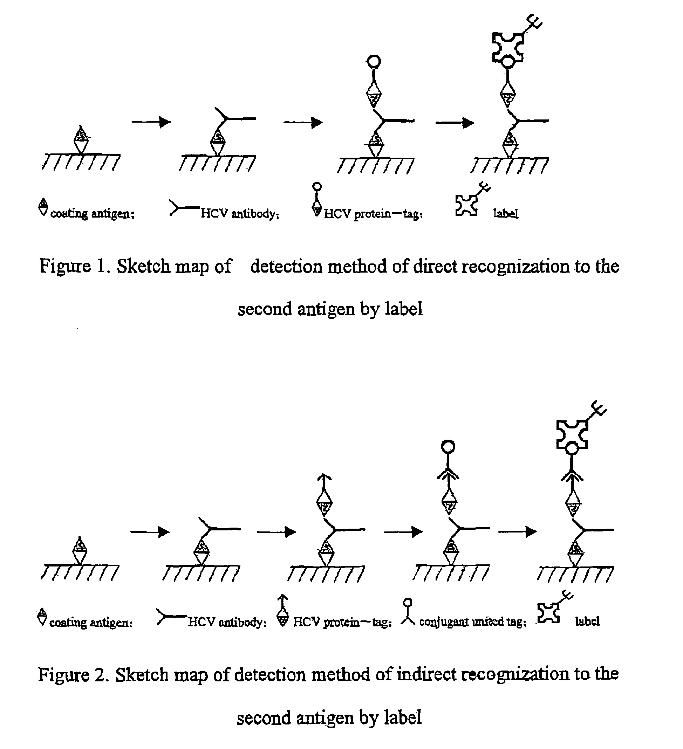

FIG.1 is a diagram depicting detecting method of label direct recognizing secondary antigen.
FIG-2 is a diagram depicting detecting method of label indirect recognizing secondary antigen.

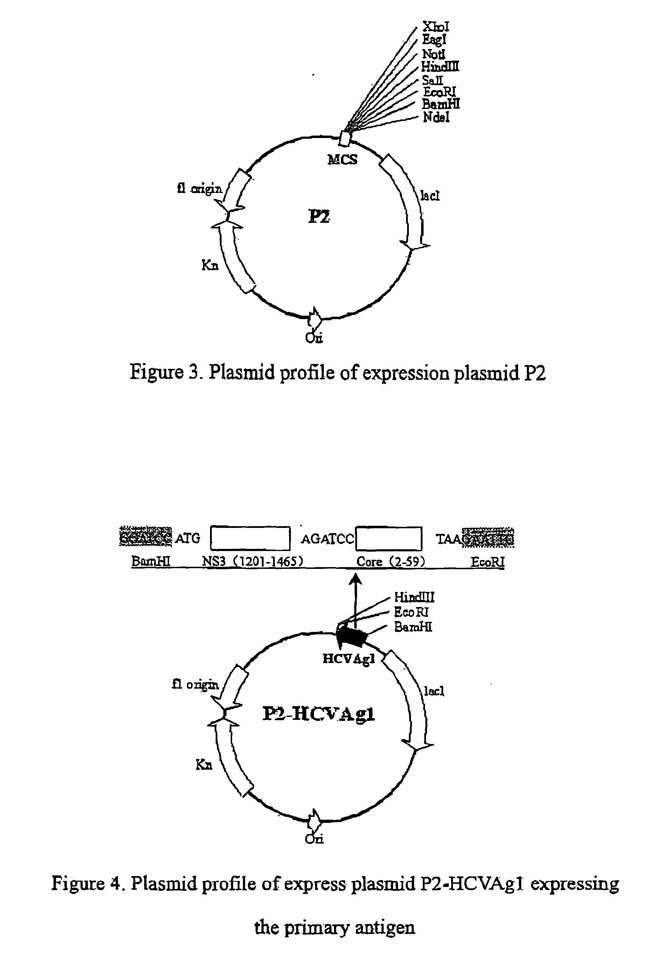

FIG.3 is a plasmid-profile depicting expression vector P2.
FIG.4 is a plasmid profile depicting expression plasmid P2-HCVAg1 of primary antigen.

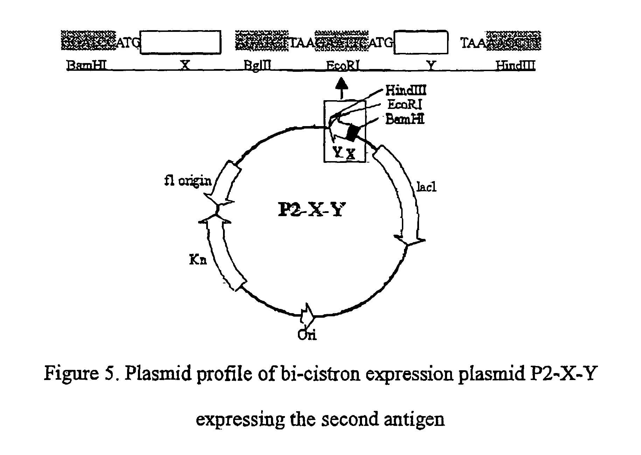

FIG.5 is a plasmid profile depicting bi-cistron expression plasmid P2-X-Y of expression secondary antigen.

### CONTENTS OF THE INVENTION

### DEFINITIONS

The following definitions serve to illustrate the different terms and expressions used in the present invention.

The term used herein 'primary antigen' refers to coating antigen in the present invention.

The term used herein 'secondary antigen' refers to conjugate of HCV protein and tag, the secondary antigen used herein can be one or several, and respective tags thereof satisfied the limited condition in the present invention.

The term used herein 'label' means the agents that are marked with detectable agents. Said detectabel agents include, but not limited to enzymes, such as horseradish peroxidase, alkaline phosphatase, β-galactosidase; chemical illuminants, such as acridinium ester; fluorescent agent, such as fluorescein isothiocyanate, europium, nanometer particle and so on; radioactive agent, such as ¹²⁵l and so on; colloid, such as gold colloid, emulsion and so on. Detectable agents can be combination of one or more above-mentioned agents in the present invention.

The term used herein 'Heterologous Protein' refers to peptide or protein moiety other than HCV protein which chimeric express with HCV protein in the present invention.

The term used herein 'biotin or derivatives thereof refers to biotin or functionality derivatives thereof, including, but are not -limited to D-biotin, activated biotin, biocytin, ethylendiamine biotin, cadaverine biotin, desthiobiotin and so on, with common characters of containing imidazolone ring or functional similar structure which can bind with avidin.

The terms used herein 'peptide' and `protein' have the common meaning as the ordinary one in the art understand, and can be used interchangeably.

The terms used herein 'CORE' and 'NS3' respectively refer to HCV core protein and non-structural protein 3. The former means the a.a.1-191 segment of protein encoded by whole gene (a.a. is amino acid simplified character, in the present invention a.a.A-B means segment from number A to number B amino acid in the HCV protein encoded by whole gene, wherein B>A), the latter means the a.a.1027-1657 segment of protein encoded by whole gene.

The term used herein 'Biotin Acceptor Protein' refers to biotin carboxyl-carrier protein (BCCP) family or functional domain thereof, SEQ ID NO.3 polypeptide or functional analogs thereof (detailed view Peter, J.S. et. al., Biotechnology, 11:1138-1143, 1993), in the present invention the biotin carboxyl-carrier protein selected can be one, several or combinations of above-mentioned peptides or proteins.

The term used herein 'Biotin-[acetyl-CoA-carboxylase] synthase' comprises enzyme whose class number is EC6.3.4.15 or domain segment and functional analogs thereof.

The term used herein 'binding partner' refers to a ligand or receptor which can bind to tag in the present invention. When choosing non-peptide tag, its binding partner are tag specific receptor or antibody; if choosing biotin or derivatives thereof or their conjugate with peptide or protein as tag, their binding partner are avidin, streptavidin, neutron-streptavidin, their analogue, antibiotin or derivatives thereof antibody; when choosing peptide as tag, their binding partner are monoclonal antibody or polyclonal antibody against the peptide or protein.

The term 'high background' refers to detected HCV negative sample's OD value exceeding normal background limit of the present invention kit, even achieving strong positive sample's OD value in normal status.

The term used herein 'flexibility' and 'rigidity' main refer to an agent structure's distortion, when macro molecule protein such as HRP etc. is cross-linked with HCV antigen, said protein can kink with HCV antigen, resulting in the latter's structure being changed, and than affect activity thereof; this kind of agent is 'flexibility' agent; inversely, when polystyrol, gold colloid etc. is cross-linked with HCV antigen, both sides will not kink together, so HCV antigen conformational changed little, this kind of agent is 'rigidity' agent.

The term used herein 'expression host' comprise prokaryotic, eukaryotic expression system host, including, but are not limited to bacterium, such as Escherichia coli, bacillus subtilis and so on: yeast such as Pichia methanolica, Saccharomyces cerevisiae and so on; eukaryotic cell such as entomo-cell, mammalian cell and so on; virus; tissue, organ, bioreactor in vivo and so on.

The term used herein 'support' include, but are not limited to ELISA plate, bead, microparticle, slide, array, plastics, membrane and so on, prepared by materials such as polystyrene, polyethylene, cellulose, nitrocellulose, acetylcellulose, silicide and so on.

The term used herein "sample/specimen to be detected' comprise all kinds of body fluid from species which can be infected by HCV, including, but are not limited to whole blood, serum, plasma, secretion, urine, saliva, cerebrospinal fluid, lymph fluid, tissue fuid, diachorema leachate, cell culture fluid or cell, tissue, organ homogenate and so on.

### TAG

The term used herein 'tag' is an agent which can link with HCV protein and specifically bind to binding partner, and furthermore educe following detected reaction process, including peptide tag and non-peptide tag.

Said peptide tag is peptide or protein, including peptide of protein containing His Tag, T7 Tag, S Tag, Flag Tag, HA Tag (many peptide can be selected, detailed see Abcam company epitope tags sections product introduction http://www.abcam.com) or HCV peptide segment et.al., said HCV peptide segments are not present in primary antigen and do not have the binding capability of causing high background, whose corresponding binding partner peptide or protein existed or can be prepared. They are characterized in that the core substance which can specifically bind to binding partner, and furthermore educe following detected reaction process' is peptide or protein. Said His Tag means six successive His, i.e. 6xHis.

Said peptide tag is Heterologous Protein, with the molecular weight smaller than 30KD, preferable smaller than 20KD, and furthermore preferable smaller than 15KD, and furthermore preferable smaller than 10KD and furthermore preferable smaller than 5KD.

Said peptide Heterologous Protein tag has a molecular weight smaller than 5KD when it is at the N-terminal of HCV protein; when said peptide tag is at C-terminal, its molecular weight is smaller than 20KD.

Said non-peptide tag is chemical compound (such as dinitrophenol, bromodeoxyuridine), hapten, vitamin (such as biotin or derivatives thereof), steroids (such as Digoxin), dyestuff (such as Acridinium Ester, Rhodamine, Dansyl Chloride, Dihydroxyfluorane, Oregon Green, Lucifer yellow, Alexa Fluor, Cascade Blue), hormones, antibiotics, nucleic acids and so on (many non-peptide can be selected, detailed see Invitrogen company molecular probe product introduction http://www.probes.com), whose corresponding binding partner non-peptide exist or can be prepared. They are characterized in that the core substance which can specifically bind to binding partner, and furthermore educe following detected reaction process is non-peptide or non-protein.

Tag can be one, several or combination of several tags, can exist at HCV antigen N-terminal, C-terminal, both terminals or in inside

Tag should be as possible as small, in order to reducing HCV antigen activity reduction.

### PREPARATION AND DETECTION PRINCIPLE OF KIT OF THE INVENTION

The present kit completes detection by the form of 'support-primary antigen-HCV antibodies to be detected-secondary antigen-label-detectable signal', wherein secondary antigen take place one or more association reaction with label, and the main preparation and detection process are as following:
Coat: coat the primary antigen on support.
Block: block the unoccupied places not coated by primary antigen on the support with inert protein, to prevent nonspecific adsorption in following reaction.
Primary antigen captures antibody: add analyte specimen and incubate to allow primary antigens to capture the specimen antibodies.
Antibody captures secondary antigen: add secondary antigens and incubate to allow HCV antibodies to capture secondary antigens.
Detect: use tag to introduce label by one or more incubating steps and then detect.

The present kit's key advantages are as following:
1. To achieve high activity label for HCV protein: to aim directly at the activity loss problem after labeling HCV protein, particular preferred is the method of biotinylating HCV protein or chimericly expressing HCV protein with peptide tag method to complete indirectly labeling of HCV protein. Because of biotin or peptide tag's molecular weight is small, after indirect labeling the antigen spatial structure is little affected, and furthermore by enzymatic reaction biotinylation the biotin can be site-directed attach to particular lysine residues in biotin acceptor protein which are chimeric with the HCV protein. Through chimeric expression with HCV protein the peptide can be site-directed inserted in certain location of HCV protein, thus HCV secondary antigen's activity can be preserved better.
2. Solve the problem of high background of HCV antibody detection agent in double antigen sandwich method: to aim directly at above-mentioned binding character of CORE antigen, high activity, weak nonspecific binding CORE antigens are screened out, solving the high background problem.
3. biotin-avidin magnifying system: raising sensitivity
   Avidin and biotin have very strong affinity, whose affinity constant (Ka: 10¹⁵ mol⁻¹) is at least 10 thousand times higher than affinity constant of antigen and antibody (Ka: 10⁵⁻¹¹ mol⁻¹), so the two agents can quickly conjugate, and the reaction are not suffer from outside interfere. The system is a high performance biological response magnifying system applied in immunologic later stage of age 70s and older. The preferred methods for preparing the present kit is to label strepto-avidin with horseradish peroxidase, recognizing biotinylated HCV secondary antigen by this label, that is to utilize biotin-avidin magnifying system as a bridge to complete the indirectly recognition of secondary antigen by label, where the system incorporation make the kit sensitivity substantially raised.
4. Three steps of specific recognition raise specificity: the three-step-reactions of the present invention's detection process are all specific recognition reactions: specimen HCV antibody specifically recognizes primary antigen; secondary antigen specifically recognizes HCV antibody; label specifically recognizes secondary antigen. This means the present invention agent's specificity is obviously improved over the traditional two steps specific recognition of double antigen sandwich agent, and obviously much better compared to the one step specific recognition in indirect method.

Moreover, the present invention detection's principle can apply to several immunoassay methods such as enzyme linked immunoassay, Luminescence Immunoassay, fluroimmunoassay, radio- immunity, microparticle immunoassay or Immunogold Assay et.al., having a broad using range.

### Preferred HCV protein regions in primary antigen and secondary antigen

In the present invention HCV genotype used is HCV 1b, and for genotype distribution information in different area different genotype segments or combination of several genotype segments can be utilized, however, the selected amino segments can keep invariably, the skilled in the art can understand it according to ordinary knowledge.

In the HCV protein, CORE and NS3 are HCV main epitope segments, the epitope regions of them located in N-terminal and C-terminal respetively, i.e. CORE's a.a.1-90 and NS3's a.a.1192-1657. After analysis and screening of aboved-mention segment, it is comfirmed that the core epitope segment thereof are CORE's a.a.7-21, 29-48, 49-90 and NS3's a.a.1359-1454. Through screening we choose a.a.1201-1465 (below short for G) to be NS3 antigen at last.

In addition, through research we find that the key segment evoking CORE protein aggregating is a.a.1-90. To solve the high background problem caused by the CORE antigen in the HCV double antigen sandwich detecting antibody kit, we design several different chimeric expressed antigens of CORE segments and NS3 G segments as primary antigen and secondary antigen, in order to screen CORE antigen gene segment scope, which can keep CORE antigen high sensitivity and also ensure their binding ability non to affect developing HCV double antigen sandwich detecting antibody kit. Also we get satisfied double antigen sandwich method HCV antigen CORE segment's characters: contain a.a.10-17 complete sequence segment, and particularly contain a.a.7-21 complete sequence segment, and particularly contain a.a.6-23 complete sequence segment; at the same time they do not contain a.a.29-90 complete sequence segment, and particularly a.a.29-70 complete sequence segment, and particularly a.a.29-59 complete sequence segment, and particularly a.a.32-48 complete sequence segment. Said protein segment can exist mutation while keeping original antigenicity and binding ability.

Above-mention rules are important rules for the present invention kit, and also for developing other HCV double antigen sandwich detecting kit which use containing CORE segment antigen.

At last we determined that primary antigen CORE segment is a.a.2-59, and secondary antigen CORE segment is a.a.1-28.

### HCV protein conjugate with tag in the secondary antigen

In the present invention lead in tag and binding partner to complete HCV protein indirect label, in choosing tag, one major criterion is that tag must as smalt as possible to reduce HCV antigen activity damaged. Tag can be one, several or combination of a single tag, can bind in HCV protein N-terminal, C-terminal, both-terminals or inside.

All exist with or can be prepared with their corresponding binding partner non-peptide agent, such as chemical compounds (dinitrophenol, bromodeo- xyuridine);vitamin (biotin or derivatives thereof); steroid (Digoxin); dyestuff (Acridinium Ester, Rhodamine, Dansyl Chloride, Dihydroxyfluorane, Oregon Green, Lucifer yellow, Alexa Fluor, Cascade Blue) and so on, or their conjugate with peptide or protein can be the present invention tag.

Peptide which all existed or can be prepared according to their corresponding binding partner peptide or protein can be the present invention tag, such as His Tag et, al..

Some micromolecule can detect chemoluminescence agent (such as Acridinium Ester), fluorescent agent (such as fluorescein isothiocyanate, europium), radioactive isotope (such as 125l) et. al., or 'rigidity' macromolecule can detected, such as gold colloid, latex particle, nanometer particle couple with label et. al., which can be prepared to label the antigen by direct label HCV protein and use in double antigen sandwich detecting. These detected agents can be a special tag to some extent in the present invention.

As for tag selecting, for this domain, common technologists can employ available empirical methods to deduct the basis for above principles, and here we no longer detail them. And here we just use His Tag as to represent peptide, use biotin or derivatives thereof as non-peptide description.
Preferred HCV protein binding with tag method of the present invention is: HCV protein biotinylation; HCV protein chimeric expression with peptide.

### 1 Biotinylation of HCV protein

Particular methods of the biotinylation of HCV protein includes:
enzymatic biotinylation in vivo;
biotinylation in vitro;
indirect biotinylation;
biotinylation of amino acid.

### 1.1 enzymatic biotinylation in vivo

The chimeric gene X encoding biotin acceptor and HCV protein and the gene Y encoding biotin-[acetyl-CoA carboxylase] synthase are mainly co-expressed in the host in the present invention, making particular lysine residues of the protein encoded by the chimeric gene X associated to biotin via enzymatic reaction in vivo with expressed biotin-[acetyl-CoA carboxylase] synthase in host, to prepare biotinylated HCV protein.

Natural biotin acceptor protein mainly exists in biotin carboxyl carrier protein family, whose core segment is the protein or peptide which containing special lysine binding to biotin. Polypeptide SEQ ID NO.3 is an artificial sequence, which has the same biotin biding ability as biotin carboxyl carrier protein. The two biotin acceptor proteins' common character is that they can bind with biotin by effect of biotin-[acetyl-CoA carboxylase] synthase, both of which can be used in this detecting system. We select SEQ ID NO.3 gene encoding products as biotin acceptor in description of the present invention.

Biotin-[acetyl-CoA carboxylase] synthase which can catalyse biotinylattion of protein have various original sources. These include, but not limited to Bacterium, such as Escherichia coli, Bacillus subtilis, Haemophilus influenzae, Rhizobium, Lactobacillus, Salmonella typhimurium, Synechocystis sp., streptococcus and Paracoccusdenitrificane BirA gene encoding products, Rickettsia mooseri BirA gene encoding products, Methanococcus jannaschii MJ1619 gene encoding products, Treponema Pallidum TP0357 gene encoding products; yeast, such as Schizosaccharomyces pombe SPBC30D10.07c gene encoding products, Saccharomyces cerevisiae BPL1 gene encoding products; plant, such as Arabidopsis thaliana BirA gene encoding products; animal, such as Homo sapiens or Mus musculus HLCS gene encoding products. Biotin-[acetyl-CoA carboxylase] synthase also includes above enzymatic domain segments or domain analogs thereof. We select BirA gene products of Escherichia coli ER2566 in description in the present invention.

### 1.1.1 Polycistron expression

It is a method to accomplish the biotinylation wherein gene X and Y were expressed in the form of polycistron in the host under the effect of the same promoter in the expression vector.

Operon is an important tissue form of prokaryotic gene expression regulatory. Most Escherichia coli gene compose gene expression regulatory unit in the form of operon. Regulated genes encoding protein in operon are structural gene. One operon has more than two structural genes, even exceeding ten. Every structural gene is a successive open reading frame, which has translation initiation code at 5-terminaland and translation termination code at 3-terminal. Air structural genes connect from the beginning to the end, arranged in series together to form structural gene groups. At least there has ribosome binding site at the 5-terminal of first structural gene, therefore, when this DNA which contain several structural genes is transcribed to polycistronic mRNA, it can be recognized and binding to ribosome, and then start translation. Ribosome move along on mRNA; after finishing the synthesis of the first coded polypeptide, ribosome can continue to translate and synthesize next gene coded polypeptide without departing from mRNA until completing the synthesis of all the polycistronic mRNA coded polypeptide.

Above-mentioned polycistron expression forms exist in many biosystem. According to above principle, we ligate X and Y in expression vector in the form of bi-cistronic, and after transformed in the expression host, attach biotin added in medium to the particular lysine sites of biotin acceptor protein chimeric expressed with HCV protein with host vivo agents and environment and biotin-[acetyl-CoA carboxylase] synthase expressed by Y, thus finish site-directed attach biotinylation of HCV protein.

The polycistrons can have X at N-terminal or C-terminal, also can be the polycistrons in the form of one X and several Y, one Y and several X, and all arranged forms of several X and several Y and so on.

In all methods of biotinylating the antigen of the present invention, 'Polycistron expression' is a method of most steadily, conveniently, and at the minimal cost; we use this method as preferred method of biotinylating antigen and attaching HCV protein with tag in the present invention.

### 1.1.2 Co-transformation expression

It is a method to accomplish the biotinylation by introducing in the expression host two different expression vectors which contain respectively gene X and gene Y simultaneously.

Gene X and Y are cloned respectively into two different expression vectors, which can have the same or different profiles, as long as they can respectively express HCV antigen with chimeric biotin acceptor protein and biotin-[acetyl-CoA carboxylase] synthase under the corresponding resistance selection pressure, and ensure that biotin-[acetyl-CoA-carboxylase] synthase can biotinylate HCV antigen maximum.

### 1.1.3 poly-promotor expression

It is a method to accomplish the biotinylation wherein gene X and Y respectively use different promoters in the same expression vector to initiate transcription and co-express in the host.

Gene X and gene Y are cloned into the same expression vector under the control of two promoters which are regulate by different operons to perform effective transcription and translation, and further to implement co-expression of HCV antigen with chimeric biotin acceptor protein and biotin-[acetyl-CoA carboxylase] synthase in the same host, and finally to finish HCV antigen biotinylation.

### 1.1.4 monocistronic expression

It is the method to accomplish biotinylation wherein gene X and gene Y are chimeric expressed in the form of monocistron in different ways,

Gene X and Y can be chimeric expressed in different forms, and the chimeric protein expressed not only has synthetics function but also has biotin acceptor ability, which can accomplish biotinylation of each other among chimeric proteins. Here said different forms can be X in N-terminal or C-terminal, also can be one X and several Y, one Y and several X, all combinations of several X and several Y and so on.

### 1.1.5 Co-expression with host biotin-[acetyl-CoA carboxylase] synthase gene

It is a method of using biotin-[acetyl-CoA carboxylase] synthase expressed by the gene similar to gene Y in expression host to biotinylate protein encoded by gene X maximum.

We use gene engineering method to incorporate endogenous or heterologous gene encoding biotin-[acetyl-CoA carboxylase] synthase into expression host, or activate biotin-[acetyl-CoA carboxylase] synthase gene endogenous to expression host in a certain manner to increase expression level thereof, resulting in biotin-acetyl-CoA carboxylase synthase expressed biotinylating fusion protein of HCV protein and biotin acceptor protein maximum. Alternatively, use the biotin-[acetyl-CoA carboxylase] synthase encoded by an expression host endogenous gene similar to Y gene to achieve the part biotinylation of HCV protein. Generally the expression host endogenous gene similar to Y gene has a low expression level in host, which results in the original host which is not engineered yet can just perform biotinylation of binding biotin protein or peptide in vivo at low efficiency. So it is required for the latter method to remove most non-biotinylated antigen, to eliminate as possible the competition of non-biotinylated antigen with biotinylated antigen when non-biotinylated antigen is used as secondary antigen in the reaction, and other biotinylation methods also need to remove non-biotinylated antigen to increase sensitivity.

### 1.2 Biotinylation in vitro

### 1.2.1 Label HCV protein with Activated biotin

Through linked-coupled method to couple activated biotin directly to HCV protein, with which HCV protein any group couple and specific activated biotin selecting and couple methods can refer to America PIERCE company catalogue.

### 1.2.2 Use enzymatic reaction in vitro to add biotin on HCV protein

Enzymatic reaction generally means to mix product encoded by X and biotin-[acetyl-CoA carboxylase] synthase in vitro and provide invariable enzymatic reaction environment, and make biotin-[acetyl-CoA carboxylase] synthase to finish HCV protein biotinylation by enzymatic reaction in vitro.

### 1.3 Indirect biotinylation

Indirect biotinylation means that HCV protein link with A agent, and use B agent to conjugate A agent, and than use C agent to conjugate B agent, the rest may be deduced by analogy to implement A first-stage or many-stage recognization, at last-stage conjugate use above-mentioned biotinylation method to link biotin, at last as HCV protein- A(-B-C......)-biotin forms to finish HCV protein indirect biotinylation.

### 1.4 Biotinylation of amino acid

Biotinylation of amino acid is to biotinylate certain or several amino acid (shenzongxuan et.al., synthetic chemistry, 10(4): 59 361, 2002) and then as host medium component it is added in expression system, and use host protein translation process to incorporate biotin into HCV protein peptide and prepare biotinylated secondary antigen.

### 2. chimeric expression of HCV protein and peptide

Except biotin tags, peptide also can be secondary antigen tag. Peptide tag can be incorporate in HCV protein N-terminal, C-terminal, amphi-terminal or internal using genetic engineering chimeric expression method. At this time tag binding partner is peptide specific reorganization agent, such as monoclonal antibody or polyclonal antibody.

Because of the specific inherence of HCV antigen, which is displayed in that the activity reduce as the heterologous protein molecular weight increasing, this appear more obviously when fuse in N-terminal, so peptide selected as tag shall has molecular weight as small as possible in the present invention.
In the present invention selecting peptide tag is His Tag.

### 2.1 Non-HCV peptide tag

Here we select non-HCV peptide as His Tag, by genetic engineering method the gene encoding His Tag and the gene coding HCV protein is chimericly expressed to prepare secondary antigen, and using label anti-His Tag monoclonal antibody to recognize secondary antigen and finish detection.

### 2.2 HCV peptide tag

When select HCV peptide as tag, must pay attention to two bellow:
1) The HCV peptide fragment should be fragment of the antibody that has no special affinity with primary antigen to prevent label binding with primary antigen.
2) The HCV peptide had better to avoid HCV protein segment with serious trend of aggregating to avoid the kit high background.

The particular preparation and detection process thereof is similar to those as above.

Through specific embodiment and further to detailed explain below.

### Example 1

### Preparation of primary antigen

The present invention get the reconstruction vector pTO-T7 referring to method of wenxin luo et al, Chinese Journal of Biotechnology, 16(5): P578-581, 2000. We synthesize primers to amplification sequence between NdeI and BamHI sites, wherein former primer has Ndel site and BamHI and EcoRI sites, and the reserve primer has BamHI site and EcoRI sites. After extracted (All molecular biology extraction and purification kits are bought from Watson Biotechnologies, Inc in the present invention), the fragment is cleaved by restriction endonuclease NdeI/EcoRI (all kinds of molecular biology enzyme are bought from TAKARA Biotechnology (Dalian) Co., Ltd. in the present invention) and ligated to the pTO-T7 vector cleaved by the same endonucleases. Positive clone is cleaved by restriction endonuclease BamHI, then extracted and ligated to each other (that is to remove T7 Tag gene which is between Ndel and BamHl sites in pTO-T7 vector), and we get the vector named P1.

Because upstream the T7 promoter of P1 vector there is one BgIII point, which is disadvantageous to clone of the present invention, so synthesis primers to amplify segments between BgIII and XbaI of P1 vector, sense primer has BamHI point and anti-sense primer has XbaI point thereof. The segment is cleave by BamHl and Xbal and then clone in P1 vector cleave by BamHI and XbaI too, and resulted positive clone is P2 expression plasmid in which BgIII point is blocked, and also is cloning vector used in the present invention. In practising the present invention, P1 and P2 are not necessary expression vectors, upstream enhancer thereof also has not substantial affection in the present invention, and construction of these vectors are just for convenience in cloning, many other expression vectors such as pET-24a(+) (America Novagen Co., Article No. 69749-3) also be used in the present invention practice.

Amplify a.a.1201-1465 gene (SEQ ID -NO.2) by PCR, sense primer thereof has BamHI point; anti-sense primer has BgIII point and EcoRI point as well as a termination codon TAA between two points. Amplify a.a.2-59 gene (the 4th to 177th nucleotide in SEQ ID NO.1) by PCR, sense primer thereof has BamHI point, anti-sense primer has BgIII point and EcoRI point as well as a termination codon TAA between two points. Clone a.a.1201-1465 gene which is cleaved by BamHI/EcoRI into P2 expression vector cleaved by the same enzyme, and obtain P2-G. Insert a.a.2-59 gene which is cleaved by -BgIII/EcoRI into the expression vector cleaved by BgIII/EcoRI, and obtain the positive clone P2-G-CORE (a.a.2-59), which is also the expression plasmid of this invention primary antigen, named P2-HCVAg1. ER2566 strain containing the plasmid has been stored in China Center for Type Culture Collection (Wuhan University, Luojia hill, Wuchang district, Wuhan, Hubei province, China) in January 21, 2005, with store number of CCTCC M 205009, and expression antigen name of HCVAg1. Clone designed in screening primary antigen in the present invention is constructed by the same way.

Transform Escherichia coli ER2566 (America New England Biolabs, Inc.) with P2-HCVAg1, and spread it in LB plate which contain 100ug/ml Kanamycin (Shanghai Sangon Biological Engineering Technology & Services Co., Ltd., below short for Sangon, Article No. KE170), stay overnight to cultivate at 37°C, and then pick separate colony to shake cultivation to OD600 approximate 1.0 in 500ml LB culture which contain the same concentration Kanamycin. Then add IPTG (Sangon, Article No. IB0168) to induce, whose final concentration is 0.5mM; the induction condition is 37°C, 200rpm and 4 hours. Harvest induced cells by centrifugation at 5000 rpm for 20 minutes and resuspend in 10 ml lysis buffer (50mM Tirs-HCl, 1 mM EDTA, 100mM NaCl, pH8.0) in per liter culture of engineering bacteria. Ultrasonic Cell, then to collect inclusion body by centrifugation at 12000 rpm for 20 minutes at 4°C, resuspend in Solution I (20mM Tirs-HCl, 5mM EDTA, 100mM NaCl, pH8.5) containing 2% Triton X-100 (Sangon, Article No. T0694). Collect inclusion body by centrifugation at 12000 rpm for 20 minutes at 4°C, dissolved by 4M carbamide in Solution I and then dialyze to 100 times voluminal PB buffer (20mM, pH7.0), by exchange 3 times PB buffer, and centrifugate to remove sediment to provide raw antigen. Equilibrate Sephacryl S-200 (America Amersham Biosciences Inc.) with the same PB buffer as above, and then add above raw antigen. Collect and pool the outflow which contains interest protein. After that, purify with CM-Sepharose (America Amersham Biosciences Inc.), adsorb HCV antigen on CM-Sepharose, elute with PBS buffer (20mM PB, 50mM NaCl, pH7.0.) to remove impurity protein not absorbed, and then elute interest protein with PBS buffer (20mM PB, 500mM NaCl, pH7.0). Finally, measure protein concentration and store at 4°C or -20°C.

### Example 2: preparation of secondary antigen

### 1. Preparation of the biotinylated secondary antigen.

### 1.1 Enzymatic biotinylation in vivo

### 1.1.1 Co-transformation and expression

Amplify a.a.1-28 gene coding HCV antigen (the 1st nucleotide to 84^{th} nucleotide in SEQ ID NO.1). The sense primer has the BamHI site and the anti-sense primer has the BgIII and EcoRI sites and a termination codon TAA between them. Amplify the gene segment L (SEQ ID NO.3) that codes the biotin acceptor protein, sense primer has BamHI point and anti-sense primer has B-gIII and EcoRI points and a termination codon TAA between the two points.

Then, ligate the sequence coding protein a.a.1-28 cleaved by restriction endonuclease BamHI/EcoRI to the expression vector P2-G cleaved by restriction endonuclease BgIII/EcoRI, get the positive clone-P2-G-CORE (a.a.1-28), named P2-HCVAg2 and the expressed antigen named HCVAg2. Ligate the gene L cleaved by endonuclease BamHI/EcoRI to the expression vector P2-G-CORE(a.a.1-28) cleaved by BgIII/EcoRI, then get the positive clone P2-G-CORE(a.a.1-28)-L named P2-X.

The biotin acceptor protein of the present invention can be biotin carboxyl carrier protein family or its function domain segments, besides peptide or domain analogs coded by SEQ ID NO.3. We design the primers to amplify the gene coding biotin carboxyl carrier protein and complete the clone using that gene to replace the gene L. The following are the primers:
Sense primer 1: 5'- CGCGGATCCATGAAACTAAAGGTAACAGTCAACGGC A -3'
Sense primer 2: 5'-GGCAGGATCCCCGGGTAAGGCAGGAGAGGGCGAGA TTC-3' Anti-sense primers:
5'-CGCGAATTCTTAAGATCTACCACCTTCTATTAACTCTAAATCCCCGATCTTGATGAG-3'

The sense primer has the BamHI site and the anti-sense primer has the BgIII and EcoRI sites as well as a termination codon TAA between them. We can get gene XaI coding segment of the biotin carboxyl carrier protein (BCCP) including 128 amino acid residues using the Pinpoint TM Xa-1 (Promega, lot number V2031) as the PCR template, sense primer1 and anti-sense primer as the PCR primers. We also can get gene Xas including 81 amino acid residue coding segment of the biotin carboxyl carrier protein using the sense primer 2 and anti-sense primer as PCR primers. Similar to gene L, the proteins coded thereof have the special lysine site which can bind the biotin. We compare the capacity that bind the biotin of the three protein and find: XaI is more powerful than Xas which is more powerful than L, that is intact and big molecular BCCP has the very powerful binding capacity, however, HCV antigen lose its activity with the increase of the molecular of BCCP. So, after biotinylation, the three proteins have the similar activity-as the secondary antigens combined to the HCV protein C-terminal. In the following experiments, we will take gene L as BCCP gene to explain the clone.

Extract total DNA of the E.coli. ER2566 as the template to amplify the BirA gene (SEQ ID NO.4) coding Acetyl-CoA Carboxylase synthase. The following is the primers:
Primer 1: 5'-CCC GAA TTC ATG AAG GAT AAC ACC GTG CC-3'
Primer 2: 5'-GGG AAG CTT TTA TTT TTC GGC ACT ACG CAG GGA TAT TTC ACC-3'

There is an EcoRl site in the sense primer and HindIII site in the anti-sense primer. The gene Y amplified is cleaved by endonuclease EcoRI/HindIII, then clone in plasmid vector pET-32a(+) (Novagen Company, lot number 70785-3) cleaved by the same endonucleases and get the new plasmid named pET-32a-Y.

The expression vector P2-X has the kanamycin resistant gene and PET-32a-Y has the ampicillin resistant gene. The two vectors are co-transformed into expression host E.Coli. ER2566. We can screen the positive clone using the double resisitant LB in which the kanamycin concentration is 100ug/ml and the ampicillin's is the same as kanamycin. Then the positive clone cultured in the 500ml LB culture medium of 100ug/ml kanamycin, 100ug/ml ampicillin, 20uM D-biotin (Sigma-Aldrich company, lot number B-4501), shaking at the temperature of 37°C to OD value to about 1.0, then induced by IPTG whose final concentration is 0.5mM, 37°C, shakes 200rpm for 4 hours. Centrifuge and collect the bacteria cells, re-suspend the cells by lysis buffer as the ratio 10ml to 1l, disrupt the cells by ultrasonic disrupter, then centrifugate for 20 minutes, at 12000rpm and 4°C, then precipitate the supemate using 10%-30% saturation ammonium sulphate and dissolve the sediment using PB buffer (100mM, pH7.0), centrifuge the solution at 12000rpm, 4°C for 10 minute to get rid of sediment, purify the protein using the affinity chromatography containing the SoftLink Soft Release Avidin (the purification approach can be referred to the brochure from Promega company, lot number V2012), elute with PB buffer (100mM, pH7.0) containing 5mM D-biotin, then we will get the purified biotinylated antigen. Then, dialyze the antigen to 100 times volume PB buffer to get rid of the free biotin for three times and centrifuge the supernate, measure the concentration of the protein, preserve it at 4°Cor -20°C for future use.

### 1.1.2 Polycistron expression

After cleaved by endonuclease EcoRI/HindIII, the gene fragment Y is ligated to the plasmid vector P2-X cleaved by the same endonuclease. Then we can get a new positive clone called P2-X-Y that is the preferred expression plasmid for second-antigen in present invention. The bacterial strain ER2566 containing the plasmid has been conserved in China Center for Type Culture Collection (Wuhan University, Luojia mountain, Wuchang district, Wuhan, Hubei province, P.R.China, 430072). The number of conservation is CCTCC M 205010. Because there is a stop codon between gene X and Y, gene X and Y compose the double-cistron under the same expression and regulation element in the plasmid P2-X-Y When screen the secondary antigen in the way of expression, we take the same way to design the clone.

Transform the P2-X-Y into E. Coli ER2566, we can get the purified antigen HCVAg by the above method.

During the biotinylation, we try to add the SD sequence between the stop coden of gene X and start coden of gene Y (all the method is normal genetic engineeering technique, you can also refer the method of Tsao, KL., Gene, 169; P59-64, 1996), to increase the expression of the gene Y, which can increase the efficiency of biotinylation and increase the sensitivity of the diagnostic kit in present invention.

### 1.1.3 Multi-promoter Expression

Clone the gene Y amplified and cleaved by endonuclease EcoRI/HindIII to the plasmid vector P1 cleaved by the same endonucleases, we can get the expression plasmid called P1-Y. We can design a pair of primers up to T7 promoter and down to the stop codon as the following:
Primer 1: 5'-GGCAGATCTTAATACGACTCACTATAGGG-3'
Primer 2: 5'-CCCAGATCTTGCTAGTTATTGCTCAGCGG-3'

The sense primer and anti-sense primer both contain the BgIII site. We can amplify the whole T7 operator element containing gene X using the plasmid P2-X as template. Then, by cloning the fragment cleaved by BgIII into expression vector P1-Y cleved with the same endonuclease, we can get the expression vector P1-X/Y containing double promoters in whichh gene X and gene Y can be transcribed and translated under the regulation of their own operator element.

The P1-X/Y is transformed into E.Coli ER2566 to perform the expression and purification of interest protein.

### 1.1.4 Monocistron Expression

Amplify the gene Y as total DNA of ER2566 as the template and the primers are the following:
Primer 1: 5'-CCC AGA TCT ATG AAG GAT AAC ACC GTG CC-3'
Primer 2: 5'-GGG GAA-TTC TTA TTT TTC GGC ACT ACG CAG GGA TAT TTC ACC-3'

The sense primer contains the BgIII site and anti-sense primer contains the EcoRI site. We clone the gene Y amplified by PCR and cleaved by BgIII/EcoRI into plasmid vector P2-X treated with same endoncleases, get the expression plasmid called P2-XY. Because the endonclease sites are BgIII and EcoRI in the 3-terminal of gene X and between the sites there is a stop codon TAA, P2-X cleaved by BgIII/EcoRI, the stop codon has been cleaved. So, the gene X and gene Y composing the monocistron that is chimeric expression gene XY.

P2-XY is transformed into E.Coli ER2566 to perform expression and purification of interest protein.

### 1.1.5 Co-expression with gene coding biotin Acetyt-CoA Carboxylase-synthase in the host

P2-X is transformed to E.Coli ER2566 to express interest protein. Use the self-expression biotin- Acetyl=CoA Carboxylase synthase of ER2566 vivo to biotinylates partly of the HCV antigen. Then we use the SoftLink Soft Release Avidin mentioned above to Affinity purification.

In above methods of enzymatic biotinylation in vivo, we try a series of methods to express HCV chimeric antigen and biotin Acetyl-CoA Carboxylase synthase in soluble form, including inducing expression under low temperature, introducing the molecular chaperones to be the up stream cistron or heterologous protein and so on, and the soluble expression can increase efficiency of biotinylation. The methods that promote the soluble expression are all mastered by normal technicians in this field, so we will not expatiate.

### 1.2 Biotinylation in vitro

### 1.2.1 biotinylating HCV antigen in vitro by coupling

Here, we will illustrate the method by labeling HCV antigen's amino group with sulfo-NHS-LC-biotin. And the following is process of labeling:
1) Take 1mg HCVAg2 antigen to dialyze to the 2M PBS (100Mm PB, 150mM NaCl, pH7.2) over night;
2) Prepare the biotin solution: dissolve 2.2mg sulfo-NHS-LC-biotin in 0.4ml ultrapure water (The water what we use in present invention is all ultrapure water), prepare it immediately before use;
3) Mix protein solution and the biotin solution at the ratio of 1:10-20, crosslink them for 2 hours at 0-4°C or for 30minutes at room temperature.
4) Dialyze the reaction solution to PB buffer containing 0.05%SDS (100mM PB, pH7.2), eliminate the free biotin.
5) Add the glycerol to 50% and store at -20°C for future use.

We can also choose the different activated biotin to label the different group of HCV antigen such as mercapto group, carboxyl. You can refer to the product catalogue of Pierce Company, USA.

### 1.2.2 In vitro biotinylation of HCV antigen by enzyme catalyst.

In the special buffer contains the ATP and D-biotin, biotin-Acetyl-CoA Carboxylase can also act the function to attach biotin on the HCV antigen with biotin acceptor protein (BCP) in the cell.

We clone the gene Y coding biotin-Acetyl-CoA Carboxylase into pET-32a(+) to express it, then purify the carboxylase by co-metal affinity chromatography column and biotinylate the HCV antigen with BCP: Add the purified HCV antigen and some biotin-Acetyl-CoA Carboxylase to biotinylation buffer containing 10mM ATP (sangon, lot AB0020), 50uM D-biotin, 50mM Bicine (Sangon, lot BB0266), 10mM MgOAc (Sangon, lot MB0326), pH 8.3 to perform biotinylation reaction for 12 hours at room temperature. After that, dialyze the antigen to PB buffer (100mM, pH 7.0) at the ratio of 1:100, change the buffer three times to remove the free biotin, centrifuge the antigen and the supernatant fluid is purified by SoftLink Soft Release Avidin resin affinity chromatography column. Then elute the column with PB buffer containing the 5mM D-biotin, we can get the biotinylated antigen. The purified antigen can be used after dialyzed.

### 1.3 indirect biotinylation

We delegate the agent A for His Tag and B for anti-His Tag monoclonal antibody (Novagen Company, USA, Lot 70796-3), and biotin on B as an example to explain the indirect biotinylation of HCV antigen.

We get the clone P2-G-CORE (a.a.1-28)-His named P2-HCVAg3 according to the above mentioned methods, express and purify it, then, get corresponding antigen called HCVAg3, in which the His represents His Tag. The same nomenclature is used for other chimeric polypeptides.

The biotin label can be realized by coupling His Tag monoclonal antibody with activated biotin as same as the method above. Enzyme labeled monoclonal antibody can specifically bind HCV antigen to realize the indirect biotinylation of HCV antigen.

We know the method of enzymatic labeling the binding partner of biotin (i.e. the streptomycin) is NalO₄ oxidation. We weigh out 10mg HRP and dissolve it in 1ml ultrapure water, slowly add the 1ml 5mg/ml NalO4 solution in ultrapure water (Sangon, lot ST1244), stir it gently at room temperature avoiding light for 40 minutes, then add 0.05ml 20% glycol (Sangon, lot E0582), stir for 40 minutes avoiding light. The mixture is dialyzed to 100mM, pH 9.51 carbonate buffer over night at 4°C avoiding light after adding 2.5mg/ml streptomycin (Sangon, lot SE497) dialyzed to 100mM, pH9.51 carbonate buffer for 2 hours. The next day, we add 0.1 ml fresh 4mg/ml NaBH₄ (Sangon, lot ST1268) solution and mix, store at 4°C for 2 hours, then put the solution into dialysis bag and dialyze to PBS buffer (150mM, pH7.4) at 4°C over night. The enzyme protective agent is added and the glycerol is also added to final concentration 50%. Then the mixture is stored at -20°C avoiding light for future use.

If the secondary antigen is biotinylation HCV protein, the detection mode of this kit is:
1) direct biotinylation mode "support-primary antigen-HCV antibody analyte -biotinylation secondary antigen -HRP labeled streptomycin-HRP substrate whose color can be the detection signal"
2) Indirect biotinylation mode ''support-primary antigen-HCV antibody analyte-secondary antigen (expression product of His Tag and HCV protein chimeric gene)-biotin labeled anti-His Tag monoclonal antibody-HRP labeled streptomycin-HRP substrate whose color can be the detection signal".

### 2. Preparation of the secondary antigen with peptide tag

We take the His Tag as the example to explain the method. In the example above, we have constructed the clone P2-HCVAg3 in which the HCV antigen's carboxyl end contains His Tag and we have prepared the antigen HCVAg3. We also try attaching His Tag at the amino end of HCV antigen, at both ends and between NS3 and CORE. They all prove to be the secondary antigen in present invention. At the same way, the biotin acceptor protein can also attach to HCV antigen using the four methods. The fusion of same tag or different tag can improve sensitivity in certain, which is certificated in this invention. All methods above about gene fusion and express are all normal technology mastered by normal technique. We are not going to elaborate here.

Peptide tag can also be the HCV peptide that is not including in the primary antigen and do not have a strong combination tendency. And the principal is the similar to peptide tag. We are not going to elaborate here.

The method of HRP labeling monoclonal antibody is similar with the one of HRP labeling streptomycin. The main difference is that the quality ratio of monoclonal antibody to HRP is 9:1-2 and the labeling time is 3-6 hours.

When the secondary antigen is expression product of peptide tag and HCV antigen chimeric gene, the detection mode of present kit is: "support- primary antigen-HCV antibody analyte-secondary antigen (expression product of His Tag and HCV protein chimeric gene)-biotin labeled anti-His Tag monoclonal antibody-HRP labeled streptomycin-HRP substrate whose color can be the detection signal"

Besides HRP, the agents that can be detected are various, for example, enzymes such as alkaline phosphatase, β-galactosidase; irradiance agents such as acridine ester; fluorescence agents such as fluorescein isothiocyanate, europium, europium nanometer granule; radioactivity agents such as ¹²⁵I; colloids such as colloidal gold, latex. Choosing different detective agent is meant to choose different immune-diagnostic method, i.e. Enzyme Linked Immunosorbent Assay, Chemiluminescence immunoassay, Fluorescent Immunoassay, Time-resolved Fluorescence Immunoassay, radioimmunoassay, Gold Labeled Immunoassay and so on. Although the methods of streptomycin or monoclonal antibody labeling those detective agent and kinds of immunoassay are different, all belong to the normal experiment methods that normal techniques should master. We are not going to elaborate here:

We try different ways to fuse the primary antigen and the secondary antigen, different expression vectors and different purification methods to increase the gap between the two antigens so as to improve the specificity of present kit. All- of the try have achieved certain results. Those methods are all normal available methods that normal techniques master. We are not going to elaborate here.

### Example 3 Screening the HCV protein fragments for the primary antigen and secondary antigen

At first, we screen the NS3 antigen fragment. According to aforementioned method, we get the clone P2-G (contain the NS3 fragment from a.a.1201 to 1465), express, purify the antigen NS3G, then detect the antigen activity using indirect ELISA. Compared with C33C (NS3 a.a.1192-1457) from patent EP-A-0450931 of Chiron Company and D26 (NS3 a.a.1207-1488) from patent US6096319 of Roche Company, we find that the specificity of NS3G is improved (data show in table 1) and sensitivity correspond. We design the primers at a.a.1201±5 and a.a.1465±8, get the clone P2-NS3 (a.a.1196-1473), P2-NS3(a.a.1206-1457). Those clones are expressed, purified and detected. We find the reactivity of those antigens is similar with NS3G. Finally, we choose fragment a.a.1201-1465 (abbreviation for G) as NS3 antigen fragment of the present invention. The methods of screening are all normal experimental methods that normal techniques should master, and you can also refer to the method of patent US6096319. So, we are not going to elaborate here.

**Table 1 Specificity comparison of NS3G, C33C and D26**

| Antigen name | Result of detecting 3000 shares clinical negative | | |
|---|---|---|---|
| | positive(person) | negative(person) | Ratio of false positive |
| C33C | 13 | 2987 | 0.43% |
| D26 | 12 | 2988 | 0.40% |
| NS3G | 6 | 2994 | 0.20% |

In order to solve the non-specificity reaction coming from CORE protein fragment, we design different clone according to aforementioned method, take the HCVAg1 as the primary antigen and use different CORE fragment as the secondary antigen. The following is the comparison result of sensitivity of different core fragment as for the secondary antigen.

**Table 2 Difference of sensitivity of different core fragment for the secondary antigen**

| Secondary antigen | sensitivity | Secondary antigen | sensitivity | Secondary antigen | sensitivity |
|---|---|---|---|---|---|
| a.a.1-23 | √ | a.a.1-21 | √ | a.a.1-17 | √ |
| a.a.6-48 | √ | a.a.7-48 | √ | a.a.10-48 | √ |
| a.a.1-6 | × | a.a.1-7 | × | a.a.1-10 | × |
| a.a.23-48 | × | a.a.21-48 | × | a.a.17-48 | × |

| | | | | | |
|---|---|---|---|---|---|
| 1) a.a.1-23 denote expression product of clone P2-G-CORE(a.a.1-23), the others are the same 2) If the sensitivity can get to the standard of commercial indirect diagnostic reagent (if there is no special explaination, the standard diagnostic reagent is Murex Anti-HCV 4.0), we represent "√", otherwise represent "×". | | | | | |

We design the same clones as to CORE fragments for the primary antigen, and get the similar sensitivity result with table 2 after detecting the HCVAg as the secondary antigen.

The result above show that for sandwiched ELISA, when HCV Core primary antigen or secondary antigen lack the sequence a.a.6-23, further lack a.a.7-21, further lack a.a.10-17, the sensitivity is lower.

We design different clones of the CORE fragment of the secondary antigen, use HCVAg1 as the primary antigen; use the same kit system to detect and get the background difference for the different CORE fragments of the secondary antigen. You can see the result in table 3

**Table 3 Difference in background of different CORE fragments of the secondary antigen**

| | | | | | |
|---|---|---|---|---|---|
| CORE | | | | | 3x(a.a.1- |
| fragment | a.a.1-90 | a.a.1-70 | a.a.1-59 | a.a.1-48 | a.a.1-2828) |
| background | ++ | ++ | ++ | ++ | - ++ |
| CORE | | | | a.a.1-28&49- | |
| fragment | a.a.32-90 | a.a.32-70 | a.a.32-48 | 70 | |
| background | ++ | ++ | ++ | ++ | |
| CORE | | a.a.49-70 | | | |
| fragment | a.a.49-70 | | | | |
| background | + | - | | | |

| | | | | | |
|---|---|---|---|---|---|
| 1) a.a.1-90 represent the expression product of clone P2-G-CORE(a.a.1-90), the others are the same 2) 3×(a.a.1-28)- represent the expression product of clone P2-G-CORE(a.a.1-28)-L-Y; 3× represent three times fusion of a.a.1-28 gene 3) a.a.49-70 represent the expression product of P2-L-CORE (a.a.49-70)-G-Y 4) ++ represent higher background, + represent high background, - represent normal background. | | | | | |

The results above show: the first epitope of HCV CORE is the sequence a.a.1-28 (abbreviation for a), the second one is a.a.32-48(abbreviation for b), and the third one is a.a.49-70 (abbreviation for c). All these epitopes have the trend of combination and b is the strongest. When there are two or more than two epitopes in the secondary antigen and the fragments are exposed completely, the background of the kit is very high.

We do the similar experiments to CORE fragment of the primary antigen. The background of kit becomes higher with the longer carboxyl end of CORE. But it is not very obvious compared to that of the secondary antigen. All the experiments tell that high background of HCV sandwiched ELISA kit is caused by the CORE fragment of the secondary antigen.

The results of those experiments above show that when the CORE fragment of secondary antigen contains a.a.32-48, further a.a.29-59, further a.a.29-70, further a.a.29-90, the background of Kit is higher.

Therefore, the character of HCV CORE protein of present invention is that it contains fragment of a.a:10-17, further a.a.7-21, further a.a.6-23 and not includes a.a.29-90, further a.a.29-70, further a.a.29-59, further a.a.32-48. The protein fragment can keep the antigenic activity and combination capacity even after mutate.

For further certificating that the high background is caused by the combination of CORE antigen, we take the following experiments:
1) Validate the homologous aggregation: we choose HCVAg1 as the primary antigen
2) Validate the heterogenous aggregation: we choose HIVgp41 antigen, syphilis Tp17 antigen, Albumin Bovine V (BSA, Shanghai Weiqun bio-technology Co., Ltd) as the primary antigen.

We choose the expression product of P2-G-CORE(a.a.1-70)-L-Y as the secondary antigen, the human HCV negative serum as analyte sample(all the HCV negative or positive serum come from healthy people), using 20% Newborn Bovine Serum(NBS), 1 %BSA, and no analyte sample (sample diluent replace the sample) for detection. The table 4 is the experiment result:

**Table 4 Corroboration experiments of aggregation trend of homologous and heterogenous CORE fragment.**

| | Primary antigen | | | |
|---|---|---|---|---|
| Analyte sample | HCVAg1 | HIVgp41 | Tp17 | BSA |
| HCV negative serum | ++ | + | + | + |
| 20%NBS | ++ | + | + | + |
| 1 %BSA | ++ | + | + | + |
| Sample diluent | ++ | + | + | + |

| | | | | |
|---|---|---|---|---|
| Note: ++ represent higher background, + represent high background | | | | |

When we only use expression product of P2-G as the primary antigen, expression product of P2-G-L-BirA or P2-G-CORE(a.a.1-70)-L-Y as the secondary antigen, the high background disappears for the former, and high background for the latter.

The results above tell that high background of HCV antibody ELISA kit (double antigens sandwiched) is caused by the trend of homologous aggregation and heterogenous aggregation of CORE antigen. The reaction mode is "Support-the primary antigen-the secondary antigen-label agent-Identifiable signal".

So, the principal of choosing the CORE fragment of HCV antigen for sandwiched ELSIA is that at the precondition guaranteeing the activity of antigen, the fragment is shorter the effect is better, and first choice is amino end.

After screening above, we can confirm that the primary antigen's CORE fragment is a.a.2-59, the secondary antigen's CORE fragment is a.a.1-28.

We further try shorting the CORE fragment of the primary antigen and the secondary antigen at the precondition guaranteeing the activity of antigen. The background of the kit has improved, and the activity of NS3 antigen has improved at a certain.

### Example 4 Determination of molecular weight of the heterologous peptide tags

To research the effect of the heterologous peptide tags to activity of the HCV antigen, the present invention designed HCV clones with peptide tags of different length in the N-terminal or C-terminal. Construction of the clones, expression and purification of protein refer to the method in the Example 1 and 2. We use the HCVAg1 as the primary antigen, the purified antigens of above clones as the secondary antigens, HRP Labeled MAb anti-His Tag as the enzyme conjugate, to detect 100 HCV positive serum which was diluted with two normal serum, compared with the indirect HCV reagent (InTec Products, Inc.(Xiamen)) and the Murex Anti-HCV 4.0 kit of Abbott Company. The result was shown in table 5 and 6.

**Table 5 The effect of N-terminal peptide tags to activity of HCV antigen**

| The number of serum positive reaction to different reagents | | | | | | |
|---|---|---|---|---|---|---|
| The secondary antigen | N-terminal peptide tags approximate MW | dilute multiple of the serum | | | | |
| | | 1 | 25 | 50 | 100 | 200 |
| HCVAg-N1 | 1KD | 100 | 100 | 98 | 96 | 80 |
| HCVAg-N2 | 2KD | 100 | 100 | 98 | 95 | 79 |
| HCVAg-N3 | 5KD | 100 | 99 | 96 | 92 | 72 |
| HCVAg-N4 | 10KD | 100 | 93 | 90 | 81 | 60 |
| HCVAg-N5 | 15KD | 100 | 89 | 80 | 62 | 41 |
| HCVAg-N6 | 20KD | 100 | 88 | 76 | 55 | 39 |
| indirect HCV reagent (InTec Products, Inc.(Xiamen)) | | 100 | 75 | 62 | 40 | 28 |
| Murex Anti-HCV 4.0 | | 100 | 85 | 73 | 50 | 37 |

Remarks:
HCVAg-N1 was expression product of P2-His-G-CORE(a.a.1-28);
HCVAg-N2 was expression product of P2- His-17-G-CORE(a.a.1-28);
HCVAg-N3 was expression product of pET-30a-G-CORE(a.a.1-28);
HCVAg-N4 was expression product of P2- His-Xas-G-CORE(a.a.1-28);
HCVAg-N5 was expression product of P2- His-Xal-G-CORE(a.a.1-28);
HCVAg-N6 was expression product of P2- His-Xas-Xas-G-CORE(a.a.1-28).

**Table 6 The effect of C-terminal peptide tags to HCV antigen**

| The number of serum positive reaction to different reagents | | | | | | |
|---|---|---|---|---|---|---|
| The secondary antigen | C-terminal peptide tags approximate MW | dilute multiple of the serum | | | | |
| | | 1 | 25 | 50 | 100 | 200 |
| HCVAg-C1 | 1KD | 100 | 100 | 99 | 97 | 84 |
| HCVAg-C2 | 2KD | 100 | 100 | 99 | 97 | 83 |
| HCVAg-C3 | 5KD | 100 | 100 | 97 | 94 | 80 |
| HCVAg-C4 | 10KD | 100 | 99 | 94 | 89 | 71 |
| HCVAg-C5 | 15KD | 100 | 96 | 90 | 81 | 61 |
| HCVAg-C6 | 20KD | 100 | 94 | 87 | 74 | 49 |
| HCVAg-C7 | 30KD | 100 | 89 | 78 | 59 | 39 |
| indirect HCV reagent (InTec Products, Inc.(Xiamen)) | | 100 | 72 | 59 | 37 | 26 |
| Murex Anti-HCV 4.0 | | 100 | 83 | 69 | 45 | 35 |

Remark:
HCVAg-C1 was expression product of P2-G-CORE(a.a.1-28)- His;
HCVAg-C2 was expression product of P2 -G-CORE(a.a.1-28)-T7- His;
HCVAg-C3 was expression product of P2 -G-CORE(a.a.1-28)- L-L- His;
HCVAg-C4 was expression product of P2 -G-CORE(a.a.1-28)- Xas- His;
HCVAg-C5 was expression product of P2 -G-CORE(a.a.1-28) XaI- His;
HCVAg-C6 was expression product of P2 -G-CORE(a.a.1-28)- Xas-Xas- His;
HCVAg-C7 was expression product of P2 -G-CORE(a.a.1-28)- Xas-Xas-Xas- His.

This experiment confirmed that the fall of activity of HCV antigen is induced by the peptide tags. Referred to the result, we can conclude that the larger heterologous peptide tags' molecular weight is, the more activity of HCV antigens falls, especially the HCV antigen's N-terminal fused by peptide tags. We confirmed this effect also occurred to the primary antigen. The present invention educed a standard of choosing fusion heterologous protein: the less molecular weight of the fusion heterologous protein, the better the molecular weight of the fused heterologous protein; and the molecular weight standard can be optimized to 30KD, 20KD, 15KD, 10KD and 5KD.

When the complex of biotin and BCCP were used as the tags of the secondary antigens, we should consider the biotin linking ability of the BCCP. To ensure biotinylation not to be effected, the molecular weight of BCCP should be as less as possible, and biotin acceptor protein molecular weight is not completly restricted by above restriction

### Example 5: Production and use of the present invention

### 1. Use biotin labeled HCV antigen as the secondary antigen

Polystyrene microtiter 96-well plates(Shenzhen Jincanhua Industry Co. Ltd.) are coated over night(4°C 24 hours) with the HCV primary antigen which is diluted by carbonate buffer (50mM, pH9.51, with 0.2‰SDS) at 100ul/well. Plates are washed twice with PBST (10mM pH7.4PB, 150mM NaCl, 0.05% Tween-20). The next day, washed twice with PBST, desiccated by patting, blocked at 37°C for two hours with 120ul block buffer which contain 30% bovine serum albumin (Beijin Yuanheng Shengma Biotechnology Institute), 8% sucrose, 5‰ casein (Sigma-Aldrich, USA, product number C-8645), 1%β-mercaptoethanol (Sangon, product lot number M0482), 150mM NaCl, pH7.4, 10mM PB, desiccated by patting, dried at 20-25°C, humidity 55%-65%. Plates were packed in dry aluminum foil bags. The coating of antigen was completed. The assay can be performed by two or there steps methods.

### 1.1 Two steps method:

The antigen-coated wells are added 50ul sample, negative (normal negative serum) or HCV positive serum, then -50ul 20mM pH7.4 PBS which contains 1 %β-mercaptoethanol, 0.5‰ TritonX-100, 20%NBS, 5‰ casein, 150mM NaCl and biotin labelled secondary antigen each well, then incubate the plate for 90 minutes at 37°C. The plate is washed five times with PBST, desiccated by patting, add 100ul 20mM pH7.4 phosphate buffer containing 1‰ casein, 20% NBS and streptavidin labeled HRP each well, incubate the plate for 30 minutes at 37°C. The plate is washed five times with PBST, desiccate the wells by patting, add 50ul color A and color B in per well respectively, wherein color A contains 0.5‰ hydrogen peroxide carbamide (Sangon, Article No. UB1753), 4.76‰ sodium acetate trihydrate, 0.9‰ acetic acid and color B contains 0.32‰TMB (Sangon, Article No. TB0514), 5mM citric acid, 0.5mM EDTA-2Na, 5% methanol, 2‰ N,N-Dimethyl formamide. Then it is developed for 30 minute at 37°C avoiding from light, add 50ul stop solution containing 2M sulfuric acid to stop reaction. After adjusting the Zero with blank control at 450nm wavelength (reference wavelength is 630nm) in ELISA, read OD value thereof. Account Cutoff Value (COV): COV=mean of negative control OD value×2.0 (If negative control OD value <0.075, account as 0.075, if negative control OD value >0.075, account as actual measure value). When analyte specimen OD value ≥COV, it will be positive; when analyte specimen OD value < COV, it will be negative.

### 1.1 There steps method:

Each well of the plate is added 50ul sample dilution buffer (20mM pH7.4 PB, contain 1%β-mercaptoethanol, 1‰TritonX-100, 20%NBS) and 50ul sample or negative or positive serum, incubated for 60 min at 37°C, then washed five times with PBST, dried by patting, then added 100ul 20mM pH7.4 PBS containing 1%β-mercaptoethanol, 0.5‰TritonX-100, 20%NBS, 5‰casein, 150mM NaCl and biotin labeled HCV antigen (diluted before use) to each well. The other steps are same to method of the two steps.

Dilution ratio of biotinylated HCV antigen and enzyme labeled streptoavidin should be groped to the best effect based on the practice. The dilution ration and each step reaction time can also be adjusted in practice production to ensure detection effect and short detection time to raise working efficiency. For the techniques of grope and adjustment are mastered by common technicians, and here we no longer detail them.

The method of three steps is preferred in this invention.

### 2. Use of His Tag labeled HCVAg3 antigen as the secondary antigen

Specific method refers to production and usage of kit that biotinylation HCV antigen is used as the secondary antigen.

### Example 6 The evaluation of the present invention kit

### (1) Sensitivity

We compare the sandwiched kit that biotinylated HCV protein is used as the secondary antigen with commercial indirect ELISA kit in sensitivity and get the similar result with table 6. The result shows that for diluted serum at various dilution level the reaction sensitivity difference between the present invention kit and the commercial indirect ELISA kit is statistics significance (P<0.05), It is evidence that, the sensitivity of present invention kit is superior to existent indirect reagent.

### (2) Specificity

Because taking three steps to specific recognition, the sandwiched kit is greatly superior to the indirect reagent of only one step specific recognition. We respectively detect 3000 shares clinical negative serum using the present invention kit and HCV indirect kit of InTec products, Inc. (Xiamen), and the ratio of false positive sera of the former is 0.067%, of the latter is 0.67%. We further use the Murex Anti-HCV 4.0 indirect ELISA kit to detect the false positive sera of the latter, and the ratio is 0.4%.

### (3) Suspicious serum

Through screening, we get bellow 12 shares suspicious sera which- is diagnosed to be positive by our own indirect reagent, to be suspicious by HCV RIBA confirmatory reagent (Singapore Genelabs Inc., Article No. 11130-018), to be negative by PCR detection (Switzerland ROCHE Inc. COBAS Ampliscreen^{™} HCV 2.0), and suppliers thereof have not any clinical hepatitis symptom (data show in tab 7). 11 shares sera are negative detected by sandwich method except C5, and these all be positive detected by indirect method using sandwich method's coating antigen and labeled antigen respectively as coating antigen of indirect method. RIBA results are that C4 are positive alone, but above two antigens both have not NS4 segment. From results above in combination with clinic, we can conclude that 9 shares sera should be false positive for own indirect reagent. C5 is similar with those 9 sera and although it shows positive in detection of sandwiched method, the OD value is obviously lower than that from detection of indirect method. So, we conclude that C5 is the common false positive of sandwiched and indirect method. In the same way, the other 10 serums except C4 and C6 are false positive of Murex Anti-HCV 4.0 kit. In the end, the kit of the present invention is more accurate than existent commercial reagent and our own indirect reagent in detecting suspicious serum.

**Tab 7 Result of detection suspicious serum (all be negative) in the present invention**

| sample | Kit of the present invention | coating antigen indirect ELISA detection | biotin labeled antigen indirect ELISA | Murex Anti-HCV 4.0 | Genelabs HCV BLOT Version 3.0 | PCR |
|---|---|---|---|---|---|---|
| C1 | - | + | + | + | NS4 + | - |
| C2 | - | + | + | + | NS4 + | - |
| C3 | - | + | + | + | NS4 + | - |
| C4 | - | + | + | - | NS3-1 + | - |
| C5 | weak + | + | + | + | NS4 + | - |
| C6 | - | + | + | - | NS3-1 + | - |
| C7 | - | + | + | + | NS4 + | - |
| C8 | - | + | + | + | NS4 + | - |
| C9 | - | + | + | + | NS4 + | - |
| C10 | - | + | + | + | NS4 + | - |
| C11 | - | + | + | + | NS4 + | - |
| C12 | - | + | + | + | NS4 + | - |

| | | | | | | |
|---|---|---|---|---|---|---|
| Note: + positive; -negative | | | | | | |

### (4) Reproducibility

240 shares of Negative and positive analyte specimens each are detected in different times, using different batches and by different operators to evaluate reproducibility of the present invention, and result shows reproducibility of negative and positive assessment result of the present invention is 100%. That indicates the detection result of the present invention has good reproducibility and assessment result is reliable.

### (5) Stability

The whole set of reagent (include coating ELISA plate, specimen dilution, enzymatic dilution, β-mercaptoethanol, biotinylation HCV antigen, negative control, positive control, color A and B, stop solution) is placed for 72 hours at 37°C, then taken out to detect sample negative and positive quality control serum at the same condition with other reagents which store at 4°C at the same time, and the result show in table 8. Experiments indicate that the present invention has good stability.

**Table 8 Experiment result of the present invention kit stability**

| store condition | linearity | CV value | mean of positive control | mean of positive control | negative and positive quality control serum |
|---|---|---|---|---|---|
| store at 4°C | 99.90% | 9% | 1.613 | 0.023 | OD value has not obviously |
| check after 72 hours at 37°C | 99.90% | 8.5% | 1.516 | 0.018 | difference in the two condition sample |

### (6) Precision

10 or more wells parallel Repeated detection is carried out to the same known positive sample in the same reaction plate, and get OD value of each well, then calculate the CV value. The CV value are all lower than 15%, which indicates the present kit has good precision.

## Claims

1. A diagnostic kit for detecting HCV antibody that complete the detection in the form of "support - the primary antigen- the antibody to be detected - the secondary antigen- label - detectable signal", wherein the secondary antigen performs bind reaction in one or more steps in the process of detection, and the secondary antigen is the conjugate of HCV protein and tag, said tag is a peptide tag or a non-peptide tag.

2. The diagnostic kit of claim 1, wherein the non-peptide is compound, half antigen, vitamin, steroid, dye, antibiotic, nucleic acid or the combination of those agent with peptide or protein.

3. The diagnostic kit of claim 2, wherein the compound is dinitrophenol or bromodeoxyuridine; vitamin is biotin or its derivatives; steroid is digoxin; dye is acridine ester, rhodamine, dansyl chloride or fluorescein.

4. The diagnostic kit of claim 1, wherein the peptide tag is the peptide or protein containing His Tag, T7 Tag, S Tag, Flag Tag, HA Tag or HCV fragment.

5. The diagnostic kit of claim 4, wherein when the peptide tag is heterologous protein, its molecular weight is less than 30KD.

6. The diagnostic kit of claim 5, wherein the heterologous protein's molecular weight is less than 20KD.

7. The diagnostic kit of claim 6, wherein the heterologous protein's molecular weight is less than 15KD.

8. The diagnostic kit of claim 7, wherein the heterologous protein's molecular weight is less than 10KD.

9. The diagnostic kit of claim 8, wherein the heterologous protein's molecular weight is less than 5KD.

10. The diagnostic kit of claim 5, wherein when the heterologous protein is at the amino end of HCV protein, its molecular weight is less than 5KD; when it is at the carboxyl end, its molecular weight is less than 20KD.

11. The diagnostic kit of claim 1-10, wherein the primary antigen and the secondary antigen include HCV NS3 and CORE protein fragment, said NS3 protein fragment is any fragment that starts from a.a.1201±5 and ends at a.a.1465±8, and can keep its antigenic activity when having mutation therein; said CORE protein fragment contain the fragment of a.a.10-17, further contain a.a.7-21, further contain a.a.6-23 and does not include fragment a.a.29-90, further not a.a.29-70, further not a.a.29-59, further not a.a32-48, the CORE fragment can keep antigenic activity and bind capacity when having mutation therein.

12. The diagnostic kit of claim 1-10, wherein the primary antigen's CORE protein fragment is a.a.2-59; the secondary antigen's CORE protein fragment is a.a.1-28; the primary antigen and secondary antigen's the NS3 protein fragment are both a.a.1201-1465.

13. The diagnostic kit of claim 1-12, wherein when tag is biotin or its derivatives, or conjugate of biotin or its derivatives with peptide or protein, its binding conjugate is avidin, streptomycin, neural chain avidin, their analog, antibody against biotin or the its derivatives; when tag is peptide or protein, its binding conjugant is the monoclonal or polyclonal antibodies against the peptide or protein.

14. A method of preparation of diagnostic kit of claim 1, including the binding step of HCV protein and tag, said binding step includes:
1) Biotinylation of HCV protein,
2) Chimeric expression of HCV protein and peptide.

15. The method of claim 14, wherein the method of biotinylation of HCV protein includes:
1) Enzymatic biotinylation in vivo;
2) Biotinylation in vitro;
3) Indirect biotinylation;
4) Biotinylation of amino acid.

16. The method of claim 15, wherein the method of enzymatic biotinylation in vivo is to co-express the chimeric gene X that codes the HCV protein and biotin receptor protein and gene Y that codes the biotin-acetyl-CoA carboxylase synthase in host cell, and in the expression host to attach biotin to particular lysine by biotin-acetyl-CoA carboxylase synthase via enzymatic reaction in vivo, particularly include:
1) polycistron expression;
2) co-transformation expression;
3) polypromoter expression;
4) monocistron expression;
5) co-expression with biotin-acetyl-CoA carboxylase synthase of host.

17. - The method of claim 15, wherein biotinylation in vitro is to biotinylate the HCV protein by coupling activated biotin or enzymatic reaction in vitro.

18. The method of claim 16, wherein biotin acceptor protein (BCCP) is biotin carboxyl carrier protein family or their functional fragment, SEQ ID NO.3 peptide or its functional analog.

19. The method of claim 16, wherein biotin-acetyl-CoA carboxylase synthase is the enzyme of EC6.3.4.15, functional fragment or functional analog thereof.
